Europäisches Patentamt

European Patent Office

Office européen des brevets

(19)

(11) Veröffentlichungsnummer: **0 228 737 B1**

# EUROPÄISCHE PATENTSCHRIFT

(12)

(45) Veröffentlichungstag der Patentschrift: 25.09.91

(21) Anmeldenummer: 86202226.6

(22) Anmeldetag: 10.12.86

(51) Int. Cl.⁵: **H01J 61/44**, H01J 61/42, H01J 61/72

(54) **UVA-Niederdruckquecksilberdampfentladungslampe für Bräunungszwecke.**

(30) Priorität: 18.12.85 DE 3544800

(43) Veröffentlichungstag der Anmeldung:
15.07.87 Patentblatt 87/29

(45) Bekanntmachung des Hinweises auf die
Patenterteilung:
25.09.91 Patentblatt 91/39

(84) Benannte Vertragsstaaten:
**BE DE FR GB NL**

(56) Entgegenhaltungen:
EP-A- 0 168 795      FR-A- 2 333 036
FR-A- 2 539 555      GB-A- 2 027 266
US-A- 2 499 307      US-A- 4 251 750

(73) Patentinhaber: **Philips Patentverwaltung GmbH**
**Wendenstrasse 35 Postfach 10 51 49**
**W-2000 Hamburg 1(DE)**

(84) Benannte Vertragsstaaten:
**DE**

Patentinhaber: **N.V. Philips' Gloeilampenfabrieken**
**Groenewoudseweg 1**
**NL-5621 BA Eindhoven(NL)**

(84) Benannte Vertragsstaaten:
**BE FR GB NL**

(72) Erfinder: **Doeckel, Ruprecht Franz Paul**
**Sternbergweg 35**
**W-2000 Hamburg 52(DE)**
Erfinder: **Lighthart, Franciscus Antonius Stephanus**
**Zwaanhoefstraat 2**
**Roosendaal(NL)**
Erfinder: **De Ridder, Adriaan Jan**
**Zwaanhoefstraat 2**
**Roosendaal(NL)**
Erfinder: **Lamboo, Theodorus Franciscus**
**Zwaanhoefstraat 2**
**Roosendaal(NL)**

(74) Vertreter: **Peuckert, Hermann, Dipl.-Ing. et al**
**Philips Patentverwaltung GmbH Wendenstrasse 35 Postfach 10 51 49**
**W-2000 Hamburg 1(DE)**

### Beschreibung

Die Erfindung betrifft eine Niederdruckquecksilberdampfentladungslampe für Bräunungszwecke, deren Lampenkolben auf der Innenseite mit zwei die Quecksilberstrahlung in hautbräunende UV-Strahlung umwandelnden Leuchtstoffen bedeckt ist, von denen einer (A) seine maximale UVA-Emission zwischen 360 und 380 nm hat.

Bekannte Lampen dieser Art (DE-OS 28 26 091 und 30 32 741) besitzen eine Leuchtstoffschicht mit einem Gemisch aus einem Leuchtstoff mit einem Emissionsmaximum im UVA-Gebiet (315 bis 400 nm) und einem zweiten Leuchtstoff mit einem Emissionsmaximum im UVB-Gebiet (280 bis 315 nm). Für Bräunungszwecke führt aber eine Bestrahlung mit UVB-Strahlen zur Erythembildung. Bereits aus diesem Grunde sollte bei Bestrahlungslampen für Bräunungszwecke die Abgabe von UVB-Strahlung möglichst vermieden werden.

Die bekannten Niederdruckquecksilberdampfentladungslampen für Bräunungszwecke, die praktisch keine oder nur noch eine sehr geringe, unschädliche UVB-Emission aufweisen, besitzen lediglich einen Leuchtstoff mit einem ausgeprägten Emissionsmaximum im UVA-Gebiet, z.B. entweder bei etwa 350 nm oder bei etwa 370 nm.

Unter der sog. weißen Bevölkerung unterscheidet man Menschen mit unterschiedlichen Hauttypen I bis IV. Der Hauttyp I ist der hellhäutige, blonde keltische Typ, während es sich bei dem Hauttyp IV um den mittelmeerischen Typ mit hellbrauner Haut und dunkelbraunen Haaren handelt. Nicht alle diese Hauttypen lassen sich durch UVA-Strahlen mit einem einzigen ausgeprägten Emissionsmaximum im UVA-Bereich gut bräunen. Die Erfindung geht auf folgende Erkenntnis zurück: Für die Hauttypen I und II sind zur Bräunung kurzwellige UVA-Strahlen besser geeignet als langwellige UVA-Strahlung. Der Hauttyp IV läßt sich mit langwelliger UVA-Strahlung besser bräunen. Den Hauttyp III kann man etwa gleich gut bis etwas besser mit Hilfe langwelliger UVA-Strahlen bräunen. Eine optimale UVA-Strahlungsquelle für alle Hauttypen gibt es bisher nicht.

Der Erfindung liegt somit die Aufgabe zugrunde, eine Niederdruckquecksilberdampfentladungslampe für Bräunungszwecke zu schaffen, die als Ultraviolettstrahlung möglichst nur UVA-Strahlung abgibt und dennoch in der Lage ist, alle oder zumindest mehrere der vier genannten Hauttypen erfolgreich zu bräunen.

Diese Aufgabe wird bei einer Niederdruckquecksilberdampfentladungslampe eingangs erwähnter Art gemäß der Erfindung dadurch gelöst, daß der andere Leuchtstoff (B) sein UVA-Emissionsmaximum zwischen 335 und 355 nm hat.

Mit einer derartigen Entladungslampe lassen sich für die verschiedenen Hauttypen fast einheitliche Bräunungszeiten erreichen, was insbesondere für gewerbliche Bräunungsanlagen von Vorteil ist.

Wenn bei der Lampe nach der Erfindung die Leuchtstoffe A und B im Gewichtsverhältnis zwischen 2,5:1 und 1:1,5 in einem Gemisch vorhanden sind, ist diese Lampe für die Bräunung aller Hauttypen I bis IV geeignet.

Bei einer speziellen Lampe zum Bräunen der Hauttypen I und II sollte das Gewichtsverhältnis A:B der Leuchtstoffe A und B in einem Gemisch zwischen 1:3 und 1:5 betragen, während bei einem Leuchtstoffgemisch lediglich zur Bräunung der Hauttypen III und IV das Mischungsverhältnis A:B vorzugsweise zwischen 5:1 und 3:1 liegt.

Gemäß einer vorteilhaften Ausführungsform der Lampe nach der Erfindung ist der Leuchtstoff (B) auf dem Lampenkolben als eine erste Teilschicht aufgebracht, ist der Leuchtstoff (A) als eine zweite Teilschicht auf der der Entladung zugewandten Oberfläche der ersten Teilschicht aufgebracht und sind die Leuchtstoffe A und B im Gewichtsverhältnis zwischen 0,5:1 und 1:5 vorhanden. In einer derartigen Lampe wird die erste Teilschicht mit dem Leuchtstoff (B) wirksam von der den Leuchtstoff (A) enthaltenden zweiten Teilschicht gegen der Lampenatmosphäre geschützt. Hierdurch kann man für den Leuchtstoff (B) auch derartige Stoffe verwenden, die sich sonst nach längerer Zeit in Lampen wegen eines größeren Rückfalls der UVA-Strahlungsmenge weniger gut verhalten. Dabei hat sich überraschenderweise gezeigt, daß diese eine Leuchtstoffdoppelschicht enthaltenden Lampen größere UVA-Strahlungsmengen liefern, als Lampen, die mit einem Leuchtstoffgemisch oder nur mit einem Leuchtstoff versehen sind.

Der Leuchtstoff (A) mit dem Emissionsmaximum zwischen 360 und 380 nm besteht vorzugsweise aus mit Europium aktiviertem Strontiumborat, z.B. der Formel $SrB_4O_7 : Eu^{2+}$ (SBE).

Der Leuchtstoff (B) mit dem Emissionsmaximum zwischen 335 und 355 nm kann aus mit Blei aktiviertem Bariumdisilikat, z.B. der Formel $BaSi_2O_5 : Pb^{2+}$ (BSP) bestehen. Geeignet hierfür sind auch Cer-Magnesium-Aluminat-Leuchtstoffe, z.B. der Formel $CeMgAl_{11}O_{19}$ (CAM).

Zur Erhöhung des Wirkungsgrades der Lampe nach der Erfindung kann zwischen Leuchtstoffgemisch und Lampenkolben eine Reflexionsschicht angeordnet sein.

Einige Ausführungsbeispiele nach der Erfindung werden nunmehr anhand der Zeichnung näher erläu-

EP 0 228 737 B1

tert. Es zeigen:

Fig. 1 eine Niederdruckquecksilberdampfentladungslampe (teilweise im Längsschnitt) mit einer Reflexionsschicht,

Fig. 2 die spektrale Verteilung des Strahlungsflusses von zwei bekannten Niederdruckquecksilberdampfentladungslampen mit jeweils einem UVA-Leuchtstoff,

Fig. 3 die spektrale Verteilung des Strahlungsflusses von Niederdruckquecksilberdampfentladungslampen mit aus den beiden Leuchtstoffen nach Fig. 1 bestehenden Leuchtstoffgemischen,

Fig. 4 die spektrale Verteilung des Strahlungsflusses von zwei weiteren bekannten Niederdruckquecksilberdampfentladungslampen mit jeweils einem UVA-Leuchtstoff,

Fig. 5 die spektrale Verteilung des Strahlungsflusses von Niederdruckquecksilberdampfentladungslampen mit aus den beiden Leuchtstoffen nach Fig. 4 bestehenden Leuchtstoffgemischen.

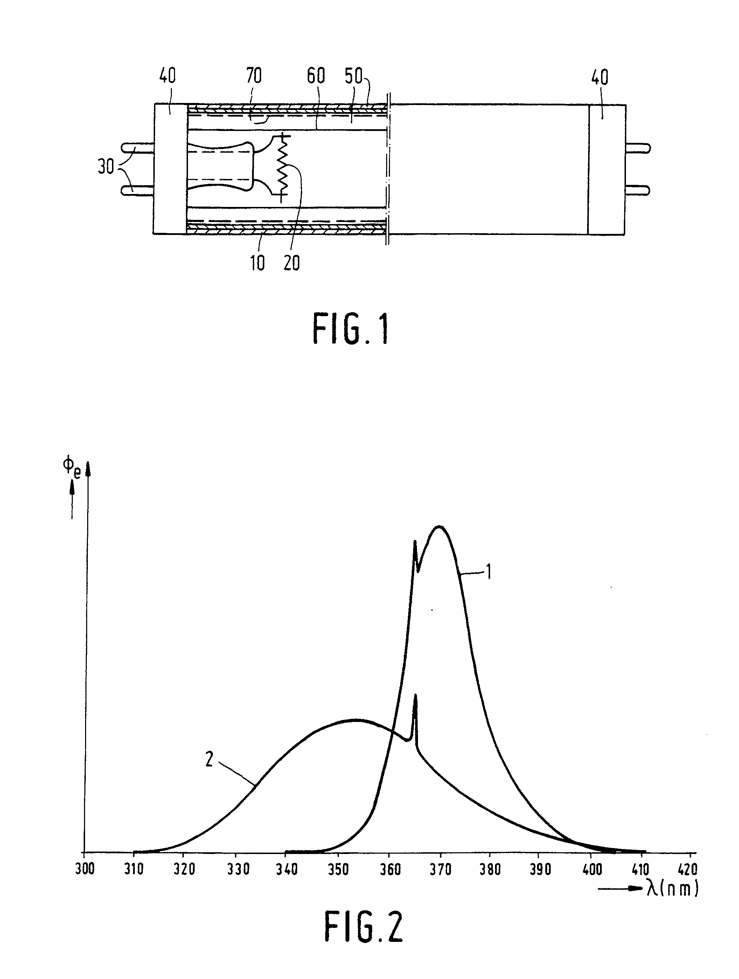

In Fig. 1 ist eine Niederdruckquecksilberdampfentladungslampe mit einem aus Natron-Kalk-Silikatglas bestehenden Lampenkolben 1O kreisförmigen Querschnittes dargestellt. In den Lampenkolben 1O ist an jedem Ende eine Wendelelektrode 2O eingeschmolzen, die jeweils mit Kontaktstiften 3O eines Sockels 4O in Verbindung stehen. Das die Entladung aufrechterhaltende Füllgas im Lampenkolben 1O ist ein Inertgas, z.B. Argon, oder ein Gemisch aus Argon und anderen Gasen bei niedrigem Druck in Kombination mit einer geringen Menge Quecksilber für den Niederdampfdruckbetrieb der Lampe. Die Innenoberfläche des Lampenkolbens 1O ist mit einer Reflexionsschicht 5O aus z.B. Aluminiumoxid bedeckt, die sich über etwa 2OO° des Kolbenumfanges erstreckt und ein Strahlungsfenster 6O frei läßt. Auf der Reflexionsschicht 5O ist eine Leuchtstoffschicht 7O angebracht, die praktisch über die volle Länge des Lampenkolbens 1O und um dessen ganzen Umfang reicht. Die Leuchtstoffschicht 7O kann aus einem Gemisch von zwei Leuchtstoffen oder aber aus zwei Teilschichten bestehen, die je einen Leuchtstoff enthalten. Das verwendete Kolbenglas wirkt als Filter, welches die UVB-Strahlung auf weniger als 1 % reduziert und UVC-Strahlung völlig ausfiltert.

Bei den für die Fig. 2 bis 5 gemessenen Leuchtstofflampen wurden folgende UVA-Leuchtstoffe verwendet:

A: mit Europium aktiviertes Strontiumborat (SBE),

B1: mit Blei aktiviertes Bariumdisilikat (BSP),

B2: Cer-Magnesium-Aluminat (CAM).

In der folgenden Tabelle sind für die gemessenen Lampen 1 bis 9 die Leuchtstoffe bzw. Leuchtstoffgemische angegeben sowie die damit erreichbare UVA-Strahlungsmenge bei 315 bis 355 bzw. bei 355 bis 4OO nm in Prozent der gesamten UVA-Strahlung.

| Lampe Nr. | Leuchtstoff (Gew.%) | | | UVA 315-355 nm in % v. UVA-Gesamtstrahl. | UVA 355-400 nm in % v. UVA-Gesamtstrahl. |
|---|---|---|---|---|---|
| | A | B1 | B2 | | |
| 1 | 100 | - | - | 2 | 98 |
| 2 | - | 100 | - | 57 | 43 |
| 3 | 80 | 20 | - | 12 | 88 |
| 4 | 60 | 40 | - | 22 | 78 |
| 5 | 20 | 80 | - | 44 | 56 |
| 6 | - | - | 100 | 55 | 45 |
| 7 | 80 | | 20 | 11 | 89 |
| 8 | 60 | | 40 | 21 | 79 |
| 9 | 20 | | 80 | 41 | 59 |

In Fig. 2 ist der Strahlungsfluß $\phi_e$ (in beliebigen Einheiten) über der Wellenlänge $\lambda$ (in nm) zweier bekannter UVA-Leuchtstofflampen mit nur einem einzigen Leuchtstoff dargestellt. Die Kurve 1 bezieht sich auf eine Lampe mit lediglich einem SBE-Leuchtstoff (A) und die Kurve 2 auf eine Lampe mit lediglich einem BSP-Leuchtstoff (B1).

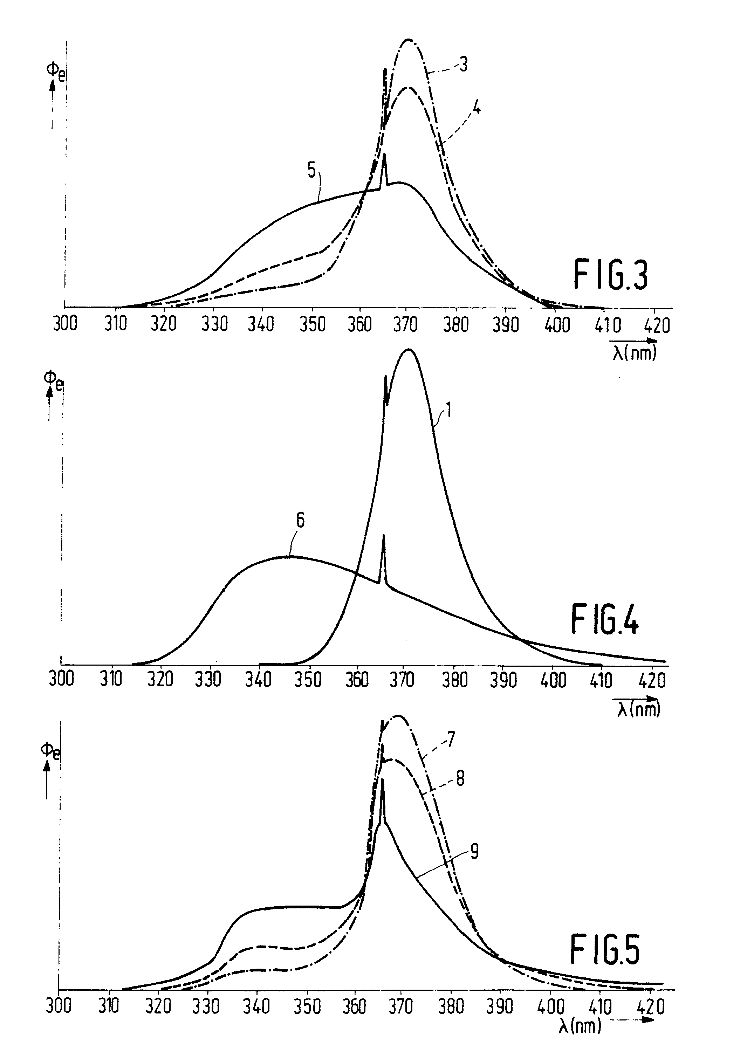

Die Kurven 3, 4 und 5 der Fig. 3 beziehen sich auf Leuchtstofflampen mit einem Gemisch eines SBE-Leuchtstoffes (A) und eines BSP-Leuchtstoffes (B1). Die Nummern der Kurven entsprechen den Lampen-Nummern in der vorstehenden Tabelle, der auch das entsprechende Leuchtstoff-Mischungsverhältnis entnommen werden kann.

3

Fig. 4 zeigt den Strahlungsfluß über der Wellenlänge zweier ebenfalls bekannter UVA-Leuchtstofflampen mit lediglich einem einzigen Leuchtstoff. Die Kurve 1 bezieht sich auf dieselbe Lampe nach Fig. 2 mit einem SBE-Leuchtstoff (A).6 ist die Strahlungskurve einer Lampe mit lediglich einem CAM-Leuchtstoff (B2).

Die Kurven 7, 8 und 9 der Fig. 5 beziehen sich auf Leuchtstofflampen mit einem Gemisch eines SBE-Leuchtstoffes (A) und eines CAM-Leuchtstoffes (B2). Die Nummern der Kurven entsprechen wiederum den Lampen-Nummern in der vorstehenden Tabelle.

Aus den Strahlungsflußkurven und der Tabelle ergibt sich, daß die Lampen 3 bis 5 und 7 bis 9 nach der Erfindung außer einem erheblichen bräunungswirksamen UVA-Anteil im Bereich von 355 bis 400 nm auch einen verstärkten UVA-Anteil im Bereich von 315 bis 355 nm aufweisen, der für die Bräunung verschiedener Hauttypen von besonderer Bedeutung ist.

Die Lampen 4 und 8 sind zur Bräunung praktisch aller Hauttypen I bis IV geeignet, die Lampen 5 und 9 insbesondere für die Hauttypen I und II und die Lampen 3 und 7 vor allem für die Hauttypen III und IV.

Es wurden Niederdruckquecksilberdampfentladungslampen von 100 W-Typ hergestellt, die auf der Innenseite des Lampenkolbens mit einer Leuchtstoffschicht (Masse 3 g pro Lampe) aus mit Blei aktiviertem Bariumdisilikat (BSP) versehen waren. Auf dieser BSP-Schicht wurde an der Entladungsseite eine zweite Leuchtstoffschicht (Masse 3 g pro Lampe) aus mit Europium aktiviertem Strontiumborat (SBE) abgelagert. Die UVA-Strahlungsmenge (315-400 nm) dieser Lampe betrug 32 W. Vergleichslampen vom selben Typ, jedoch mit einer einzigen Leuchtstoffschicht aus 6 g BSP bzw. 6 g SBE vesehen, ergaben UVA-Strahlungsmengen von 30 W.

**Patentansprüche**

1. Niederdruckquecksilberdampfentladungslampe für Bräunungszwecke, deren Lampenkolben auf der Innenseite mit zwei die Quecksilberstrahlung in hautbräunende UV-Strahlung umwandelnden Leuchtstoffen bedeckt ist, von denen einer (A) seine maximale UVA-Emission zwischen 360 und 380 nm hat, dadurch gekennzeichnet, daß der andere Leuchtstoff (B) sein UVA-Emissionsmaximum zwischen 335 und 355 nm hat.

2. Lampe nach Anspruch 1, dadurch gekennzeichnet, daß die Leuchtstoffe A und B im Gewichtsverhältnis zwischen 2,5:1 und 1:1,5 in einem Gemisch vorhanden sind.

3. Lampe nach Anspruch 1, dadurch gekennzeichnet, daß die Leuchtstoffe A und B im Gewichtsverhältnis A:B zwischen 1:3 und 1:5 in einem Gemisch vorhanden sind.

4. Lampe nach Anspruch 1, dadurch gekennzeichnet, daß die Leuchtstoffe A und B im Gewichtsverhältnis zwischen 5:1 und 3:1 in einem Gemisch vorhanden sind.

5. Lampe nach Anspruch 1, dadurch gekennzeichnet, daß der Leuchtstoff (B) auf dem Lampenkolben (10) als eine erste Teilschicht aufgebracht ist, daß der Leuchtstoff (A) als eine zweite Teilschicht auf der der Entladung zugewandten Oberfläche der ersten Teilschicht aufgebracht ist und daß die Leuchtstoffe A und B im Gewichtsverhältnis zwischen 0,5:1 und 1:5 vorhanden sind.

6. Lampe nach einem der Ansprüche 1 bis 5, dadurch gekennzeichnet, daß der Leuchtstoff (A) mit dem Emissionsmaximum zwischen 360 und 380 nm aus mit Europium aktiviertem Strontiumborat besteht.

7. Lampe nach einem der Ansprüche 1 bis 5, dadurch gekennzeichnet, daß der Leuchtstoff (B) mit dem Emissionsmaximum zwischen 335 und 355 nm aus mit Blei aktiviertem Bariumdisilikat oder aus einem Cer-Magnesium-Aluminat besteht.

8. Lampe nach einem der Ansprüche 1 bis 7, dadurch gekennzeichnet, daß zwischen den Leuchtstoffen (70) und dem Lampenkolben (10) eine Reflexionsschicht (50) angeordnet ist.

## Claims

1. A low-pressure mercury vapour discharge lamp for tanning purposes, whose envelope is coated on the inside with two luminescent materials which convert the mercury radiation into skin-tanning UV radiation, one of which (A) has its maximum UVA emission between 360 and 380 nm, characterized in that the other luminescent material (B) has its UVA emission maximum between 335 and 355 nm.

2. A lamp as claimed in Claim 1, characterized in that the luminescent materials A and B are present in a mixture in a weight ratio of between 2.5 : 1 and 1 : 1.5.

3. A lamp as claimed in Claim 1, characterized in that the luminescent materials A and B are present in a mixture in a weight ratio of between 1 : 3 and 1 : 5.

4. A lamp as claimed in Claim 1, characterized in that the luminescent materials A and B are present in a mixture in a weight ratio of between 5:1 and 3:1.

5. A lamp as claimed in Claim 1, characterized in that the luminescent material (B) is applied to the lamp envelope as a first layer, the luminescent material (A) is applied as a second layer to the surface of the first layer facing the discharge, and that the luminescent materials A and B are present in a weight ratio of between 0.5 : 1 and 1 : 5.

6. A lamp as claimed in any one of Claims 1 to 5, characterized in that the luminescent material (A) having its emission maximum between 360 and 380 nm consists of europium-activated strontium borate.

7. A lamp as claimed in any one of Claims 1 to 5, characterized in that the luminescent material (B) having its emission maximum between 335 and 355 nm consists of lead-activated barium disilicate or of cerium-magnesium aluminate.

8. A lamp as claimed in any one of Claims 1 to 7, characterized in that a reflective coating (50) is provided between the luminescent materials (70) and the lamp envelope (10).

## Revendications

1. Lampe à décharge dans la vapeur de mercure à basse pression pour le bronzage comportant une ampoule dont la face intérieure est recouverte de deux substances luminescentes transformant le rayonnement du mercure en rayonnement ultra-violet de bronzage, l'une (A) des substances luminescentes présentant son émission UVA maximale entre 360 et 380 nm, caractérisée en ce que l'autre substance luminescente (B) présente son maximum d'émission UVA entre 335 et 355 nm.

2. Lampe selon la revendication 1, caractérisée en ce que les substances luminescentes A et B sont présentes dans un mélange dans un rapport en poids compris entre 2,5 : 1 et 1 : 1,5.

3. Lampe selon la revendication 1, caractérisée en ce que les substances luminescentes A et B a sont présentes dans un mélange dans un rapport en poids A : B compris entre 1 : 3 et 1 : 5.

4. Lampe selon la revendication 1, caractérisée en ce que les substances luminescentes A et B sont présentes dans un mélange dans le rapport en poids compris entre 5 : 1 et 3 : 1.

5. Lampe selon la revendication 1, caractérisée en ce que la substance luminescente (B) sur l'ampoule de lampe (10) est appliquée sous forme d'une première couche partielle, la substance luminescente (A) est appliquée sous forme d'une deuxième couche partielle sur la surface de la première couche partielle située vis-à-vis de la décharge et les substances luminescentes A et B sont présentes dans un rapport en poids compris entre 0,5 : 1 et 1 : 5

6. Lampe selon l'une des revendication 1 à 5, caractérisée en ce que la substance luminescente (A) dont le maximum d'émission se situe entre 360 et 380 nm est constituée par du borate de strontium activé à l'aide d'europium.

5

7. Lampe selon l'une des revendications 1 à 5, caractérisée en ce que la substance luminescente (B) dont le maximum d'émission se situe entre 335 et 355 nm est constituée par du disilicate de baryum activé à l'aide de plomb ou par un aluminate de magnésium-cérium.

8. Lampe selon l'une des revendications 1 à 7, caractérisée en ce qu'une couche de réflexion (50) est appliquée entre les substances luminescentes (70) et l'ampoule de lampe (10).

FIG.1

FIG.2

FIG.3

FIG.4

FIG.5